Europäisches Patentamt

**(19)** European Patent Office

Office européen des brevets

**(11)** **EP 0 949 507 A1**

**(12)** **EUROPEAN PATENT APPLICATION**
published in accordance with Art. 158(3) EPC

**(43)** Date of publication:
**13.10.1999 Bulletin 1999/41**

**(21)** Application number: **97949254.3**

**(22)** Date of filing: **26.12.1997**

**(51)** Int. Cl.[6]: **G01N 33/50**, G01N 33/53

**(86)** International application number:
**PCT/JP97/04885**

**(87)** International publication number:
**WO 98/29745 (09.07.1998 Gazette 1998/27)**

**(84)** Designated Contracting States:
**CH DE ES FI FR GB IT LI NL SE**

**(30)** Priority: **26.12.1996 JP 35644596**

**(71)** Applicant:
**FUJI YAKUHIN KOGYO KABUSHIKI KAISHA
Takaoka-shi Toyama 933-8511 (JP)**

**(72)** Inventors:
 • **OBATA, Ken-ichi,
Fuji Yakuhin Kogyo K.K.
Toyama 933-8511 (JP)**
 • **SAKAI, Tomoe,
Fuji Yakuhin Kogyo K.K.
Toyama 933-8511 (JP)**
 • **HIRANO, Kayoko,
Fuji Yakuhin Kogyo K.K.
Toyama 933-8511 (JP)**
 • **YOSHIDA, Shin-ichi,
Fuji Yakuhin Kogyo K.K.
Toyama 933-8511 (JP)**
 • **IWATA, Kazushi,
Fuji Yakuhin Kogyo K.K.
Toyama 933-8511      11 (JP)**

**(74)** Representative:
**von Kreisler, Alek, Dipl.-Chem. et al
Patentanwälte,
von Kreisler-Selting-Werner,
Bahnhofsvorplatz 1 (Deichmannhaus)
50667 Köln (DE)**

**(54)** **AGENT FOR TREATING URINE SPECIMEN**

**(57)** Urine precipitates are frequently formed upon urine storage and the precipitate is a big obstacle for conducting an accurate assay and for sampling a large number of samples because minor constituents, such as analytes to be measured by immunoassay, biochemical assay, etc., are possibly incorporated into the precipitate. The present invention provides an agent for treating urine for solving the problem of the precipitate formed during storage of the urine and also provides a method therefor. When urine is treated with an agent for treating the urine sample where said agent contains a buffer in the pH range of 6.5 or more, the precipitate which is formed by storing the urine can be solubilized. Said precipitate can also be solubilized by making the pH of urine 6.5 or more. In addition, when the precipitate formed by storing the urine is solubilized or said formation is inhibited or prevented, it is now possible to subject a large number of samples to assay by an easy operation even on a mass scale whereby a correct and clinically significant result can be obtained.

FIG. 1

## Description

Technical Field

The present invention relates to agents for treating a urine sample, said agents which are utilized in the field of immunoassay, biochemical assay, etc. and allow us to obtain more accurately and easily assay results. The urine sample-treating agent according to the present invention enables us to solubilize minor constituents which are potentially incorporated into precipitates formed during the storage of urine and further to transport urine samples in a substantially stable state and/or keep them in a good state of long-term preservation, from the viewpoint of immunoassay, biochemical assay, etc.

Background Art

Urine contains end products and intermediate metabolites of protein, nucleic acid metabolism as well as various organic and inorganic salts and is useful for diagnosis of various diseases and for understanding of physiological states via grasping the quantitative or qualitative changes of those substances. Immediately after urination, urine is clear but, when it is stored and allowed to stand, various salts may be precipitated to cause turbidity even in normal urine. When a target substance to be analyzed (an analyte) is incorporated into precipitates formed as such, an accurate quantitative assay is not achievable unless an adverse influence caused by the precipitate is eliminated, for example, by dissolving the precipitate.

In order to use the urine as a sample in immunoassay, biochemical assay, etc. and to obtain more correct and reliable results, it is unavoidable to eliminate the formation of such a precipitate or to remove the bad influence by the precipitate. Moreover, in the field where immunoassay, biochemical assay, etc. is carried out, the means which is mostly conducted in a test department, a test center, etc. having various measuring instruments and reagents with an object of handling many targets (items) to be measured where fresh urine is not always used is becoming important recently rather than the test for very limited objects (items) to be measured using fresh urine (where only simple test is usually possible and the analytes which are targets for the assays are therefore limited).

Now, although urine has a difficult problem in view of transportation and storage, it has an advantage that, unlike blood, it can be collected easily and on a mass scale without damaging the living body and, as mentioned already, when final products and intermediate metabolites of protein, nucleic acid metabolism as well as various organic and inorganic salts contained therein are assayed and tested, that will be greatly helpful in diagnosis of disease and in understanding the state of health of organism and the state of intake of the pharmaceuticals. In that case, the collected urine (sample) is usually preserved by cooling or by freezing, sent to a test department, a test center, etc. and stored there. However, it has been noted that the above-mentioned precipitate is frequently generated when the urine is preserved with cooling or freezing.

Especially in the clinical area of recent years, most of quantitative assays for specific substances in urine are carried out in the above-mentioned test department, test center, etc. and, therefore, it is difficult to use a urine immediately after urination where no precipitate is generated. Since precipitate is generated in the urine which is sent to the test department, test center, etc., it is necessary for a correct assay of the specific substances to fully suspend the precipitate followed by sampling and that is quite troublesome. Especially when many samples are requested to be treated, the work of fully suspending the precipitate generated in the sample is quite laborious and is a neck for automation of the work as well. When suspension of the precipitate is insufficient, sampling error is resulted which may affect the clinical evaluation and that is a big problem in view of reproducibility as well.

Thus, in order to treat a large number of urine samples in correct and efficient manners with saving the labor and to obtain clinically significant data, it is essential to solve the problem of precipitate generated in urine.

According to "Rinsho Kensa-ho Teiyo (Handbook of Clinical Test Methods)" (29th Edition), pages 108-109 (1983), urine may be turbid by precipitation of various salts even in a normal state and, in an alkaline case (urine from a person taking much vegetable food or urine after taking a meal), urine is often turbid due to phosphates or carbonates even immediately after urination while, in a concentrated acidic urine, large amount of precipitate in a brick-like pink color may be generated upon cooling. That consists of urates and disappears upon warming. There is a disclosure in the above reference concerning the methods for differential diagnosis of urinary cloudiness for the precipitates generated in urine such as urate, carbonate, phosphate, calcium oxalate, uric acid, pus, fat and bacterial urine, and, with an object of the differential diagnosis, urine is heated or admixed with acetic acid, hydrochloric acid or sodium hydroxide to check the precipitate in urine. However, in said document, there is no consideration at all by taking the utilization as a urine sample to be used in immunoassay, biochemical assay, etc. and, in urine which is treated for the differential diagnosis of urinary cloudiness as such, denaturation of protein and big change in pH of urine take place whereby it is difficult to use such a urine in immunoassay, biochemical assay, etc. Moreover, with a view for obtaining clinically significant data, it is believed that there is no recognition for solving the problems of precipitation in urine samples at all in said document.

As mentioned above, the urine sample is an important thing in a medical field, such as clinic and therapy, and also in a hygienic field for measuring and for detecting various final products and intermediary metabolites in protein, nucleic acid and carbohydrate metabolism as well as various organic and inorganic salts and pharmaceuticals. For further utilization of such urine samples in future, it will be essential that the samples are sent to test department, test center, etc. equipped with sufficient facilities and staffs, stored and then utilized for the test. For such a purpose, it has been demanded that the bad affection caused by the precipitate which is generated upon storage, freezing or cooling of the urine sample is solved in a reliable and safe manner. Up to now, the only method available is that the urine sample is suspended which, however, is time-consuming, troublesome and unreliable and there has been a demand for offering an art by which correct clinical test results can be ensured by a simple and reliable method instead of the above-mentioned prior art one.

Disclosure of the Invention

The present inventors have conducted an extensive investigation with an view that, if precipitates usually formed in urine samples can be made soluble while suppressing the denaturation of proteins included in urine as much as possible without greatly changing the pH of urine, it will be possible to easily carry out urine sampling, to eliminate a variation in test results which normally depend on how to sample and accordingly to achieve reliable and correct assays. As a result, the present inventors have succeeded in completing the present invention.

An object of the present invention is to solubilize minor constituents potentially incorporated into the precipitate in urine to such an extent that an accurate and reliable quantitative assay is not disturbed substantially for specific substances in urine.

Another object of the present invention is to allow us to make urine sampling suitable for immunoassays, biochemical assays, etc., to adjust assay operations to automation, and to easily and reliably acquire clinically significant data regardless of forming precipitates in the urine sample.

The present invention provides:

(1) an agent for treating a urine sample (i.e., "urine sample-treating agent"), which comprises, as an effective component, a buffer in the pH of 6.5 or higher;

(2) the urine sample-treating agent according to the above (1) which comprises, as an effective component, a buffer in the pH range of from 6.5 through 9.5;

(3) the urine sample-treating agent according to the above (1) or (2) which comprises, as an effective component, a buffer in the pH range of from 6.5 through 8.2;

(4) the urine sample-treating agent according to any of the above (1) to (3) which comprises, as an effective component, a buffer in the pH range of from 7.2 through 8.0;

(5) the urine sample-treating agent according to any of the above (1) to (4) which comprises, as an effective component, a buffer in the pH range of from 7.4 through 7.8;

(6) the urine sample-treating agent according to any of the above (1) to (5) which comprises, as an effective component, a buffer in the pH range of from 7.4 through 7.6; and

(7) the urine sample-treating agent according to any of the above (1) to (6) in which the buffer is a member selected from the group consisting of phosphate buffer, N-tris(hydroxymethyl)methyl-2-aminoethanesulfonic acid (TES) buffer, N-(2-hydroxyethyl)piperazine-N'-ethanesulfonic acid (HEPES) buffer, tris(hydroxymethyl)aminomethane (Tris)-HCl buffer, borate buffer, citrate buffer, barbital buffer and imidazole-HCl buffer.

In another aspect, the present invention provides:

(8) an agent for treating a urine sample, which comprises the property of solubilizing at least part of precipitates formed in urine;

(9) the urine sample-treating agent according to the above (8) in which the urine is for a sample used in immunoassays and/or biochemical assays;

(10) the urine sample-treating agent according to the above (8) or (9) in which the "solubilizing" includes dissolving at least part of precipitates formed in the urine sample so as not to have substantially an adverse effect on assays;

(11) the urine sample-treating agent according to any of the above (8) to (10) which comprises the property of solubilizing minor constituents which are or may be potentially incorporated into precipitates formed in the urine sample and are analytes to be assayed;

(12) the urine sample-treating agent according to any of the above (8) to (11) in which the precipitate formed in the urine sample occurs during the storage of the urine sample;

(13) the urine sample-treating agent according to any of the above (8) to (12) in which the precipitate formed in the urine sample occurs during the storage of urine in the pH of lower than 7.0;

(14) the urine sample-treating agent according to any of the above (8) to (13) in which the precipitate formed in the urine sample occurs during the storage of urine in the pH range of from pH 5.0 through 6.8;

(15) the urine sample-treating agent according to any of the above (8) to (14) in which the "solubilizing" is carried out by increasing the pH of urine with the buffer according to any of the above (1) to (7);

(16) the urine sample-treating agent according to any of the above (8) to (15) in which the "solubilizing" is carried out by adding tris(hydroxymethyl)aminomethane (Tris)-HCl buffer to urine;

(17) the urine sample-treating agent according to any of the above (8) to (16) in which the minor constituent is selected from the group consisting of urinary transferrin, urinary growth hormone, urinary IgG, urinary albumin, urinary chorionic gonadotropin, urinary $\alpha_1$-microglobulin, urinary $\beta_2$-microglobulin, various types of collagen, proline hydroxylase, laminin, matrix metalloproteinases (MMPs), tissue inhibitors of metalloproteinase (TIMPs) and thrombomodulin; and

(18) the urine sample-treating agent according to any of the above (8) to (17) in which the minor constituent is selected from the group consisting of type IV collagen and thrombomodulin.

In still another aspect, the present invention provides:

(19) an agent for treating a urine sample, which comprises the property of making the pH of urine 6.5 or higher;

(20) the urine sample-treating agent according to the above (19) which comprises the property of making the pH of urine from 6.5 through 9.5;

(21) the urine sample-treating agent according to the above (19) or (20) which comprises the property of making the pH of urine from 6.5 through 8.2;

(22) the urine sample-treating agent according to any of the above (19) to (21) which comprises the property of making the pH of urine from 7.2 through 8.0;

(23) the urine sample-treating agent according to any of the above (19) to (22) which comprises the property of making the pH of urine from 7.4 through 7.8;

(24) the urine sample-treating agent according to any of the above (19) to (23) which comprises the property of making the pH of urine from 7.4 through 7.6;

(25) the urine sample-treating agent according to any of the above (19) to (24) in which the property is achieved by (i) increasing the pH of urine or (ii) maintaining a predetermined pH value in urine, with the buffer according to any of the above (1) to (7);

(26) the urine sample-treating agent according to any of the above (19) to (25) in which the property is achieved by adding tris(hydroxymethyl)aminomethane (Tris)-HCl buffer to urine;

(27) the urine sample-treating agent according to any of the above (19) to (26) in which urine is fresh or stored;

(28) the urine sample-treating agent according to any of the above (19) to (27) in which urine is subjected to the "solubilizing" according to any of the above (8) to (18), thereby (i) substantially preventing the formation of precipitates occurring in a fresh urine from having an adverse effect on assays for target analytes, or (ii) substantially eliminating a disturbance caused by precipitates in a stored urine in view of the assays; and

(29) the urine sample-treating agent according to any of the above (19) to (28) which has the property (i) of preventing the formation of precipitates occurring during the storage of a fresh urine or (ii) of solubilizing precipitates in a stored urine.

In yet another aspect, the present invention provides:

(30) a container for a urine sample, which contains the urine sample-treating agent according to any of the above (1) to (29) and is in a form ready to use;

(31) the container according to the above (30) in which the urine sample-treating agent is contained in the form of a liquid composition;

(32) the container according to the above (30) in which the urine sample-treating agent is contained in the form of readily soluble solids;

(33) the container according to the above (32) in which the urine sample-treating agent is contained in the form of lyophilized solids; and

(34) the container according to any of the above (30) to (33) which is portable.

In still another aspect, the present invention provides:

(35) a method for treating a urine sample, which comprises treating urine with the urine sample-treating agent according to any of the above (1) to (29);

(36) the method according to the above (35) which comprises

(a) treating urine with the urine sample-treating agent, and

(b) (i) solubilizing at least part of precipitates usually formed in the urine sample or (ii) suppressing or preventing at least partially the formation of said precipitates, for the purpose of having substantially no adverse effect on assays;

(37) the method according to the above (35) or (36) which comprises either (i) solubilizing substantially all of precipitates formed in urine or (ii) suppressing or preventing entirely the formation of said precipitates, for the purpose of having substantially no adverse effect on assays;

(38) an assay for a urine sample, which comprises

(a) treating urine with the urine sample-treating agent according to any of the above (1) to (29), and
(b) then using the treated urine as a sample for assays; (39) the assay according to the above (38) which comprises

(a) treating urine with the urine sample-treating agent so as to (i) solubilize at least part of precipitates usually formed in the urine sample or (ii) suppress or prevent at least partially the formation of said precipitates, for the purpose of having substantially no adverse effect on assays, and
(b) then assaying said treated urine sample for a target analyte;

(40) the assay according to the above (38) or (39) in which, in the assay of urine samples, sampling is made and target analytes are assayed without subjecting the urine sample to suspension (or agitation);

(41) the assay according to the above (38) to (40) in which the target analyte is selected from the group consisting of type IV collagen and thrombomodulin and urine is used as a sample; and

(42) the assay according to the above (38) to (41) which comprises automatically sampling the urine and assaying for a target analyte.

In further an aspect, the present invention provides:

(43) an agent for treating a urine sample, which comprises the property of making urine from weakly acidic through alkaline so as to (i) solubilize precipitates formed in the urine sample or (ii) suppress or prevent the formation of precipitates in the urine sample;

(44) the urine sample-treating agent according to the above (43) in which the precipitate in the urine sample is or may be potentially formed during the storage of the urine sample;

(45) an agent for treating a urine sample, which comprises the property (i) of solubilizing at least part of precipitates formed during the storage of urine or (ii) of keeping at least part of components in fresh urine a solubilized state during the storage of urine, for the purpose of having substantially no adverse effect on assays;

(46) the urine sample-treating agent according to the above (45) which has the property (i) of solubilizing minor constituents which are incorporated into precipitates formed in the urine sample or (ii) of keeping said minor constituents a solubilized state during the storage of urine;

(47) the urine sample-treating agent according to the above (46) in which the minor constituents which are incorporated into precipitates formed in the urine sample are target analytes to be assayed; and

(48) the urine sample-treating agent according to any of the above (45) to (47) in which the phrase "having substantially no adverse effect on assays" means that there is a significant correlation between (i) the data obtained by assay of fresh urine and (ii) the data obtained by assay of stored urine wherein the stored urine is subjected to the assay after storing (a) at room temperature for three hours or longer or (b) at 10 °C for ten hours or longer.

In still further an aspect, the present invention provides:

(49) a method for treating a urine sample, which comprises

(a) making urine from neutral through weakly alkaline with an agent for treating the urine sample,
(b) (i) solubilizing precipitates formed in the urine sample or (ii) suppressing or preventing the formation of precipitates occurring in the urine sample, and
(c) then using the treated urine sample for assays;

(50) a method for treating a urine sample, which comprises

(a) treating the urine sample with an agent for treating the urine sample, and
(b) (i) solubilizing at least part of precipitates which are usually formed in the urine sample or (ii) suppressing or preventing at least partially the formation of precipitates occurring in the urine sample, for the purpose of having substantially no adverse effect on assays;

(51) the method according to the above (49) or (50) which comprises (i) solubilizing substantially all of precipitates formed in the urine sample or (ii) suppressing or preventing entirely the formation thereof, for the purpose of having substantially no adverse effect on assays;

(52) the method according to any of the above (49) to (51) which comprises treating the urine sample with the urine sample-treating agent according to any of the above (1) to (29);

(53) an assay for a target analyte in a urine sample, which comprises

(a) making urine from neutral through weakly alkaline with an agent for treating the urine sample,

(b) (i) solubilizing precipitates formed in the Urine sample or (ii) suppressing or preventing the formation of precipitates occurring in the urine sample, and

(c) then assaying;

(54) an assay for a urine sample, which comprises

(a) treating the urine sample with an agent for treating the urine sample, and

(b) (i) solubilizing at least part of precipitates which are usually formed in the urine sample or (ii) suppressing or preventing at least partially the formation thereof, for the purpose of having substantially no adverse effect on assays, and

(c) then subjecting said treated urine sample to the assay for a target analyte;

(55) the assay according to the above (53) or (54) which comprises treating the sample with the urine sample-treating agent according to any of the above (1) to (29); and

(56) the assay according to any of the above (53) to (55) in which urine collected in the container according to any of the above (30) to (34) is used as a sample.

In other aspects, the present invention provides:

(57) a kit which is a set comprising a combination of

(1)

(i) a reagent for the assay of human type IV collagen containing an anti-human type IV collagen monoclonal antibody or

(ii) a reagent for the assay of human thrombomodulin containing an anti-human thrombomodulin monoclonal antibody, with

(2)

(a) the urine sample-treating agent according to any of the above (1) to (16) and (19) to (29) or

(b) the container according to any of the above (30) to (34);

(58) the kit according to the above (57) which is a set comprising a combination of

(1) a reagent for the assay of human type IV collagen containing an anti-human type IV collagen monoclonal antibody with

(2)

(a) the urine sample-treating agent according to any of the above (1) to (16) and (19) to (29), or

(b) the container according to any of the above (30) to (34);

(59) the kit according to the above (58) in which the human type IV collagen assay reagent contains two kinds of monoclonal antibodies, each of which recognizes a different site on the human type IV collagen molecule from the other;

(60) an assay reagent for (i) human type IV collagen or (ii) human thrombomodulin wherein (i) the human type IV collagen assay reagent contains an anti-human type IV collagen monoclonal antibody and (ii) the human thrombomodulin assay reagent contains an anti-human thrombomodulin monoclonal antibody, which comprises, as a constituent element,

(a) the urine sample-treating agent according to any of the above (1) to (16) and (19) to (29), or

(b) the container according to any of the above (30) to (34);

(61) a human type IV collagen assay reagent containing anti-human type IV collagen monoclonal antibody, which comprises, as a constituent element,

(a) the urine sample-treating agent according to any of the above (1) to (16) and (19) to (29), or

(b) the container according to any of the above (30) to (34); and

(62) the human type IV collagen assay reagent according to the above (61) in which the reagent contains two kinds of monoclonal antibodies, each of which recognizes a different site on the human type IV collagen molecule from the other.

The phrase "making urine from weakly acidic through alkaline" used herein means that, in the measurement of an analyte (target to be assayed, such as type IV collagen, thrombomodulin, etc.), the pH of a normal urine sample (typically, pH 5.0 through 6.3) is made 6.5 or higher with the object of having substantially no adverse effect on assays for target analytes. Preferably the phrase "making urine from weakly acidic through alkaline" used herein refers to getting the pH of conventionally collected urine adjusted to from 6.6 or higher up to 8.2. Particularly, the phrase "making urine from weakly acidic through alkaline" used herein preferably refers to getting the pH of conventionally collected urine adjusted to from 7.0 through 8.2. Especially, it may mean that the pH of urine samples is made 7.5 or a near value. In another embodiment, the phrase "making urine from weakly acidic through alkaline" used herein means that, in the measurement of an analyte (target to be assayed, such as type IV collagen, thrombomodulin, etc.), a buffer is added to a urine sample (such as fresh urine and stored urine including frozen urine and cooled urine) with the object of having substantially no adverse effect on assays for target analytes so that the pH of ordinary urine samples (typically, pH 5.0 through 6.3) is made 6.6 or higher. Preferably, the phrase "making urine from weakly acidic through alkaline" used herein refers to adding a buffer to a urine sample (such as fresh urine and stored urine, including frozen urine and cooled urine) so as to get the pH of ordinarily collected urine sample adjusted to from 7.0 through 8.2. It may also mean that the pH of urine is made 7.5 or an adjacent value so as to (i) solubilize precipitates therein or (ii) have no formation of the precipitates. For analytes to be assayed, the phrase "not to have substantially an adverse effect on assays", or "having substantially no adverse effect on assays", means that a significant clinical result or a significant assay result is given. The phrases "having substantially no adverse effect on assays", etc. may also mean that there is substantially a significant correlation between (1) the data obtained by the assay of fresh urine and (2) the data obtained by the assay of stored urine including cases where the assayed, stored urine has been maintained (a) at room temperature for three hours or longer or (b) at 10°C for ten hours or longer.

Brief Description of the Drawings

Fig. 1 is a correlation in the measured IV·C values between (1) buffer (Tris-HCl)-added and (2) untreated (i.e., merely suspended) urine samples, from healthy persons, wherein precipitates were formed after being frozen, stored and thawed.
Fig. 2 is a correlation in the measured IV·C values between (1) buffer (Tris-HCl)-added and (2) untreated (i.e., merely suspended) urine samples, from diabetic patients, wherein precipitates were formed after being frozen, stored and thawed.

Best Mode for Carrying Out the Invention

In accordance with the present invention, methods for treating urine (such as a urine sample to be assayed) which comprise adding an agent for treating a urine sample (i.e., "urine sample-treating agent", including a buffer) to the urine are provided. To be more specific, methods for treating the urine with a urine sample-treating agent which contains, as an effective component, a buffer in the pH of 6.5 or higher, are provided. The present invention further provides methods for solubilizing precipitates in urine (such as a urine sample to be assayed). More particularly, it provides methods for solubilizing at least part of precipitates formed in the urine sample for the purpose of having substantially no adverse effect on assays.
In another embodiment of the present invention, methods of adjusting the pH of urine to 6.5 or higher are provided. More particularly, methods of adjusting the pH of a urine sample to from 7.0 through 8.2, or methods of keeping the urine sample in the pH range of from 7.0 through 8.2, are provided. In accordance with the present invention, agents for treating a urine sample (i.e., "urine sample-treating agents") which have such function and/or action are also provided. In accordance with the present invention, containers for collecting and/or receiving urine (i.e., "urine receptacles") where a urine sample-treating agent, such as a buffer, is contained in advance so as to subject the urine to a predetermined treatment immediately after urine collection, are provided. In another aspect, the present invention provides methods where the pH of urine is made from weakly acidic through alkaline (particularly, from neutral through weakly alkaline) so that urinary precipitates formed during the storage are dissolved and minor constituents potentially incorporated into the urinary precipitate are solubilized. The present invention also provides agents for treating urine samples (i.e., "urine sample-treating agents") which have such functions and actions. The present invention further provides containers for collecting and/or receiving urine (i.e., "urine receptacles") where a urine sample-treating agent, such as a buffer, is contained in advance so as to keep the pH of urine from weakly acidic through alkaline (particularly, from neutral through weakly alkaline) immediately after urine collection.

In accordance with the present invention, weakly acidic through alkaline substances are used for keeping the pH of urine from weakly acidic through alkaline. The weakly acidic through alkaline substances may include inorganic compounds such as weakly acidic or alkaline inorganic salts, organic compounds such as weakly acid or alkaline organic salts, mixtures thereof, etc. In order to make the pH of urine neutral, there is a method of neutralizing it with an acid or alkali; in that case, however, it is necessary to neutralize it along with checking the pH for each sample and, therefore, such a method is not so convenient for a practical purpose. On the other hand, a buffer is preferred in view of keeping the pH of urine weakly acidic through alkaline, particularly neutral through weakly alkaline, more preferably pH 6.5 through 9.0, because the object can be achieved merely by keeping the final concentration of buffer constant. Thus, in accordance with the present invention, weakly acidic through alkaline buffers may be preferably used for keeping the pH of urine weakly acidic through alkaline. Preferably buffers in the pH range of from 6.5 through 9.5 (more preferably from 7.0 through 8.0, still more preferably from 7.2 through 7.8, most preferably from 7.4 through 7.6) can be used in adjusting the urine to from pH 6.5 through 9.0 (more preferably from pH 7.0 through 8.2, still more preferably from pH 7.2 through 7.8, most preferably from pH 7.4 through 7.6). In an embodiment of the present invention, it is appropriate to use a buffer in the pH range of from 6.5 through 9.5, more preferably from 7.0 through 8.0, still more preferably from 7.2 through 7.8, most preferably from 7.4 through 7.6, in order to adjust the pH of urine to from 6.5 through 9.0, more preferably from 7.0 through 8.2, still more preferably from 7.2 through 7.8, most preferably from 7.4 through 7.6.

The buffers as used herein include those which exert a buffering action under weakly acidic through weakly alkaline conditions. Said buffers are preferably those which do not have an adverse effect on assays for target analytes wherein immunoassay and/or biochemical assay should be applied to the analyte to be measured. The buffers may be any among those which have been commonly used or can be easily used in immunoassays and/or biochemical assays in the art, as long as they are capable of making the urine sample weakly acidic through weakly alkaline (particularly preferably neutral through weakly alkaline). Examples of the buffer are phosphoric acid or phosphate buffer, tris(hydroxymethyl)aminomethane (Tris) buffer, N-(2-hydroxyethyl)piperazine-N'-(2-ethanesulfonic acid) (HEPES) solution, piperazine-N,N'-bis(2-ethanesulfonic acid) (PIPES) solution, 3-(cyclohexylamino)-1-propanesulfonic acid (CAPS) solution, 3-(N-morpholino)propanesulfonic acid (MOPS) solution, N,N'-bis(2-hydroxyethyl)-2-aminoethanesulfonic acid (BES) solution, N-tris(hydroxymethyl)methyl-2-aminoethanesulfonic acid (TES) solution, N-(2-acetamido)-2-aminoethanesulfonic acid (ACES) solution, borate buffer, citrate buffer, barbital buffer, imidazole-HCl buffer, etc. Each buffer may be used either alone or in a composition form wherein it is optionally admixed with any of other buffers.

Further, a suitable acid and/or alkali may used in a form wherein the pH can be adjusted to a suitable value, optionally either in a combination or in an admixture. The acid includes an inorganic acid such as hydrochloric acid, sulfuric acid, nitric acid, phosphoric acid, an organic acid such as acetic acid, citric acid, maleic acid, and fumaric acid, and so on. The alkali includes an alkali hydroxide such as sodium hydroxide, potassium hydroxide, sodium carbonate, potassium carbonate, sodium bicarbonate, and potassium bicarbonate, an organic base such as triethylamine, and so on.

Preferably, the buffer is selected from the group consisting of phosphate buffer, N-tris(hydroxymethyl)methyl-2-aminoethanesulfonic acid (TES) buffer, N-(2-hydroxyethyl)piperazine-N'-ethanesulfonic acid (HEPES) buffer, tris(hydroxymethyl)aminomethane (Tris)-HCl buffer, borate buffer, citrate buffer, barbital buffer and imidazole-HCl buffer. Preferred examples thereof are such buffers which preferably contain, as an effective component, a buffer element (or a set of buffer elements) in the pH range of 6.5 or higher, more preferably in the pH range of from 6.8 through 9.5, still more preferably in the pH range of from 7.0 through 8.0, further preferably in the pH range of from 7.2 through 7.8, and still further preferably in the pH range of from 7.4 through 7.6. Especially preferred examples of the buffer are Tris-HCl buffer and the like. The Tris-HCl buffer preferably includes those in the pH range of from 6.5 through 9.5, more preferably those in the pH range of from 7.0 through 8.0, still more preferably those in the pH range of from 7.2 through 7.8 and further preferably those in the pH range of from 7.4 through 7.6.

There is no particular limitation for the concentration of each buffer component so far as a sufficient buffering action is achieved. For example, it is from 0.05 through 10.0 moles, preferably from 0.1 through 5.0 moles and more preferably from 1.0 through 2.0 moles. The pH of buffer is, as mentioned hereinabove, 6.5 or more, preferably from 6.8 through 9.5, more preferably from 7.0 through 8.0, still more preferably from 7.2 through 7.8 and further more preferably from 7.4 through 7.6. A 1.5 mole (M) Tris-HCl buffer in the pH of 7.5 may be used suitably.

Since normal urine and disease urine are acidic in many cases, it is necessary to admix with an alkaline buffer, preferably a weakly acidic through alkaline buffer, in particular preferably a buffer in the pH range of from 6.5 through 9.5, for the purpose of adjusting the pH of urine to from weakly acidic through alkaline, particularly from neutral through weakly alkaline. In the case of alkaline urine, in order to dissolve precipitates formed therein, it is necessary to admix with a weakly alkaline through acidic buffer, in particular preferably a buffer in the pH range of from 6.5 through 8.2, for purpose of adjusting the pH of urine to from weakly acidic through alkaline, particularly from neutral through weakly alkaline. In admixing with the urine, the buffer may be employed in the form of a liquid or solution, in the form of a semi-solid, such as gel, or further in the form of a solid. For example, the buffer may be in the form of powders, tablets, capsules, etc. Preferred buffers are in an easily-soluble form. The buffer may also be in the form of lyophilized solids, derived from solutions. It is also possible to admix the urine with a mixture of source materials for buffers. The pH of buffers is appropriately 7 through 8. It is preferred that the buffer is contained in the urine sample-treating agent of the present invention in such a form and/or state as to exert a sufficient buffering action on a predetermined amount of urine (wherein the action may include buffering actions where time and temperature factors are dependently or independently taken into consideration).

It is also possible to contain the buffer in advance in a container for collecting and/or receiving urine (i.e., "urine receptacle"). Further, the buffer solution can be added to each urine receptacle, and then lyophilized to provide buffer solids which are stuck to the inner wall of the receptacle whereby the buffer can be mixed with the urine sample simultaneously with collecting urine. The use of such a solid buffer leads to escaping from the dilution of minor constituents in urine. The urine sample receptacle which may be constituted as such is of an instantly usable form which is capable of not only receiving fresh urine but also taking action to prevent or inhibit the formation of precipitates in the urine during storage. Said urine sample receptacle may be equipped with (a) a urine sample-treating agent in the form of a solution or (b) a urine sample-treating agent in the form of easily soluble solids, such as a urine sample-treating agent in the form of lyophilized solids. Preferred containers are those suitable for transporting and/or storing the urine sample for each person and preferably portable. Examples of such containers are those made of glass, plastics (e.g., PET, etc.) and the like. Shapes of said container may be selected from those which are commonly well known in the art, those which are conventionally used in the art, or from those which can be easily used in the art. Particularly preferred shapes are those suitable for transporting and/or storing liquids, including a tubular shape (for example, a test tube). Such shapes may include (1) those having a round bottom, and (2) those in the form of a Spitz (which has a pointed or thin end, like a tip). Preferably, said container is equipped with a tightly closing means such as a stopper. According to the present invention, fresh urine is collected in said urine sample receptacle with the result that the formation of precipitates in urine can be substantially prevented, said precipitates which have an adverse effect on assays for analytes to be measured. Further, stored urine (e.g., cooled and stored urine, freezed and stored urine, etc.) is transferred to said urine sample receptacle with the result that a disturbance caused by precipitates formed in urine can be substantially eliminated in the assays. It can now be easily carried out to collect the fresh urine in said urine sample receptacle for the purpose of preventing the formation of precipitates in urine during storage or to receive the stored urine in said urine sample receptacle for the purpose of dissolving precipitates in the urine.

A urine sample usually has a pH of lower than 7.0 and, in many cases, it has a pH range of from 5.0 through 6.9. When such a urine sample is stored (especially when stored with cooling or after freezing), it has been observed that precipitates are formed and analytes are incorporated into such precipitates. In the present invention, the buffer is added to the urine sample to adjust the pH of urine to from weakly acidic through weakly alkaline, particularly from 6.8 through 8.2, preferably from 7.0 through 7.8, more preferably from 7.2 through 7.7, and, most preferably from 7.4 through 7.6, whereupon minor constituents incorporated into the urine precipitates would be solubilized and an accurate quantitative assay for each minor constituent in urine is now achievable. The "solubilizing" of the precipitates may refer to those capable of solubilizing part of said precipitates, provided that it causes no problem in the assay, or preferably those capable of dissolving the minor constituents in said precipitate. Preferred examples of said minor constituents are analytes to be assayed.

In accordance with the present invention, it is preferred that none of components which will adversely affect and/or disturb the assay of target analytes are admixed with the urine sample-treating agent of the present invention. In order that the urine sample can be used for various assays, it is also desired to avoid anything which will needlessly affects substances to be assayed, such as analytes. For example, enzyme inhibitors, proteolysis inhibitors, chelating agents, etc. have been known to have various actions on biocomponents in the urine sample. Therefore, in utilizing the urine sample for various assays, it is desired to be free from admixing with such components if at all possible. In the present invention, the urine sample-treating agents which are free from adding enzyme inhibitors, proteolysis inhibitors, chelating agents, etc. to the urine sample, are preferred. According to the present invention, with a view to treating the urine so that the urine sample can be widely applied for various tests and assays, it is preferred that benzamidinium chloride, ethylenediamine tetraacetic acid (EDTA), sodium azide, etc. are not added to urine. Accordingly, the urine sample-treating agents of the present invention which do not contain such components are preferred.

The target analytes to be assayed include objects to be examined or assayed by immunoassays and/or biochemical assays. Examples of the target analytes are hormones, vitamins, physiologically active substances, pharmaceuticals, proteins, saccharides, metabolites thereof, and so on. Representative examples thereof are protein components contained in blood, such as urinary transferrin, urinary growth hormone, urinary IgG, urinary albumin, urinary chorionic gonadotropin, urinary $\alpha_1$-microglobulin, urinary $\beta_2$-microglobulin, type IV collagen and various types of collagen, proline hydroxylase, laminin, stromal collagenase (MMP-1), gelatinase A (MMP-2), stromelysin-1 (MMP-3), MMP-7, neutrophil collagenase (MMP-8), galatinase B (MMP-9), stromelysin-2 (MMP-10), stromelysin-3 (MMP-11), collagense-3 (MMP-13), MMP-14 (MT1-MMP), MMP-15 (MT2-MMP), MMP-16 (MT3-MMP), MMP-17 (MT4-MMP), MMP-20 (enamelysin) and other matrix metalloproteinases (MMPs), tissue inhibitors of metalloproteinase (TIMPs) (e.g., TIMP-1, TIMP-2, TIMP-3, TIMP-4, etc.), and thrombomodulin.

Urine is collected upon urination and is utilized as a sample for immunoassays, biochemical assays, etc. The pH of urine is ordinarily from about 5.0 through 6.8, more specifically from about 5.3 through 6.4. With regard to the ratio of urine in the pH of lower than 7.0 to that in the pH of 7.0 or higher, it has been reported for healthy persons (about 80 samples) that the urine in the pH of lower than 7.0 occupies about 90% and the remaining about 10% is in the pH of 7.0 or higher. In addition, it has also been reported for diabetic patients (about 10 samples) that the urine in the pH of lower than 7.0 occupies about 90% and the remaining about 10% is in the pH of 7.0 or higher. Further, among the healthy persons (about 80 samples), from the lowest to the highest pH values of urine are within the range of about 5.06 through 7.52, while those are within the range of about 5.60 through 7.10 among diabetic patients (about 10 samples).

After urine is collected upon urination, its storage often leads to the formation of precipitates therein (as also pointed out in pages 108 to 109 of the above "Rinsho Kensa-ho Teiyo", 29th Edition). For instance, it was observed that, when urine was allowed to stand at room temperature for three hours, precipitation was noted in about 56% of samples (as checked in 64 cases) and, when it was allowed to stand overnight at 10°C (stored with cooling), precipitation was noted in about 77% of samples (as checked in 64 cases).

Since precipitates are easily formed in urine as such, it is believed that, as a result of the occurrence of a precipitation in case where much time is consumed from urine collection until urine assays (including the case where various assays are to be carried out) and/or in case where samples are sent to a test center, etc. while being preserved with cooling, minor constituents in the urine will be incorporated thereinto. Especially for precipitates formed in the case where acidic urine is preserved (e.g., refrigerated urine, stored frozen urine, etc.), target analytes (to be assayed) as aforementioned are incorporated thereinto, etc. whereby an adverse effect on assays will be resulted.

In processes from urination until quantitative assays for a target component, plural dispensing and pouring operations are usually carried out. Thus, prior to the quantitative assay, (1) a distribution of urine to each transportable test tube from a cup for collecting the urine, (2) a distribution from the transportable test tube for transporting the sample to an assay place where each assay item is examined, (3) a sampling at the assay place, and so on, are ordinarily conducted. When target components incorporated into urine precipitates are present upon conducting the above-mentioned dispensing (pouring) and sampling operations, sufficient suspending operation (such as agitation) is required for each of the operations including distribution and sampling. If non-uniformity occurs upon such suspending operations, an accurate quantitative assay becomes impossible for the target component. Since the suspending operation is mechanical, however, it is considerably difficult to achieve a sufficient and uniform suspension. This difficulty is an obstacle to the automation of assays. When the aforementioned dispensing (pouring) and sampling operations are carried out for samples on a mass scale and a diversity of assays are also conducted even for a great number of samples, then there are drawbacks that they are too much troublesome.

In the present invention, a simple operation enables us to keep the final concentration of the buffer constant therein, said simple operation which is (a) an admixture of a predetermined amount of urine with the buffer in a predetermined rate or (b) an addition of a predetermined amount of urine to the container in which a predetermined amount of buffer is contained. For example, in assaying type IV collagen (IV・C) or thrombomodulin (TM) in urine, IV・C or TM which is incorporated into precipitates in urine can be made soluble when 0.1 part by volume of 1.5M Tris-HCl buffer, pH 7.5, is added to 1 part by volume of urine. As such, it is now possible to assay the minor constituents in urine easily and accurately by merely applying the readily-practicable sample-treating method thereto, without being affected by the urine precipitate. Accordingly, agents for treating a urine sample which are designed so as to achieve such an object and containers for collecting urine which contain said urine sample-treating agent are provided herein.

Further, in order to achieve that (1) plural dispensing operations can be conducted, (2) various tests, assays and analyses can be applied to urine samples, (3) dispensing and sampling operations can be carried out for a large number of samples on a mass scale and/or (4) the urine samples can be treated in assay departments, test centers, etc. wherein various measuring instruments and reagents are placed and various types of tests and assays are conducted for many samples, it is believed that urine should be sufficiently admixed with the component(s) of the present invention for avoiding the affection by the precipitate in urine, and that it is desired to avoid the addition of unnecessary components. It is also preferred that IV・C or TM can be efficiently assayed and measured by the use of urine samples, via avoiding problems accompanied by precipitates in urine and/or the formation thereof. It is particularly preferred that such an object can be achieved without adding any of benzamidinium chloride, EDTA, sodium azide, etc. to urine. Consequently, the present invention preferably provides agents for treating a urine sample which are designed for achieving such an object, containers for collecting urine which contain said urine sample-treating agent, methods for treating urine, etc.

Examples

Described below are examples of the present invention which are provided only for illustrative purposes, and not to limit the scope of the present invention. In light of the present disclosure, numerous embodiments within the scope of the claims will be apparent to those of ordinary skill in the art.

Example 1

(1) Assay of Urinary IV・C

(a) IV・C in urine is assayed by one-step sandwich EIA using two kinds of monoclonal antibodies, each of which recognizes a different site on IV・C molecule from the other.

Thus, IV・C in the sample, mouse anti-human IV・C monoclonal antibody-bound beads and enzyme-labeled mouse anti-human IV・C monoclonal antibody were made to react simultaneously to form triplet complexes thereof. Then the amount of enzymes coupled to the antibody-bound beads via IV・C was measured using, as a substrate, hydrogen peroxide in the presence of a coloring agent. Since the amount of enzymes depends upon the concentration of IV・C in the sample, the IV・C concentration in the sample can be determined from a calibration curve generated in advance using standard solutions where each IV・C content is known. For the assay reagent herein, for example, a IV・C assay kit (Panassay IV・C; Fuji Chemical Industries, Ltd., Japan) can be used.

(b) An aliquot (100 µl) of a urine sample (if precipitated, it was suspended and then collected) was dispensed to each test tube. Similarly, a standard solution (100 µl) was also dispensed to each test tube. Then 300 µl of an enzyme-labeled mouse anti-human IV・C antibody was added to the test tube, followed by well mixing. Then one mouse anti-human IV・C monoclonal antibody-bound bead was placed in each test tube, thereby leading to an antigen-antibody reaction. The antigen-antibody reaction was carried out by allowing each test tube to stand at 4 to 15°C for 24 hours. After completion of the antigen-antibody reaction, the reaction solution was removed by suction and 3.5 ml of washing solution were then added followed by removal with suction. The operation was repeated once again. Then the bead was transferred to a fresh test tube. After a coloring solution (300 µl) was added thereto, a substrate solution (100 µl) was added at predetermined intervals, then mixed, and allowed to stand at 15 to 30°C for 60 minutes, thereby leading to an enzymatic reaction. A stopping solution (1,000 µl) was added at predetermined intervals to stop the enzymatic reaction. Absorbance at 450 nm is measured wherein water is used as a control.

(2) Transfer of Urinary IV・C to Precipitate

With regard to the urine where precipitates were formed, it was centrifuged. The resultant supernatants and precipitates after the centrifugation were assayed for IV・C with a IV・C assay kit (Panassay IV・C; Fuji Chemical Industries, Ltd., Japan), respectively.

Remarks: The precipitates separated out by the centrifugation were subjected to the assay after dissolving in a diluting solution for a standard (attached to the IV・C assay kit) wherein the diluting solution was added in the same amount as that of the urine used for the centrifugation.

The IV・C was measured according to a method as mentioned in "Rinsho Kensa, Kiki-Shiyaku (Clinical Tests, Instruments and Reagents)", Vol. 18, pp. 439 to 444 (1995).

The urine where precipitates were formed was suspended and then used as a control.

The results are shown in Table 1.

Table 1

| | | | | IV・C: ng/ml |
|---|---|---|---|---|
| Urine | Supernatant | Precipitate (PPT) | Ratio (PPT/Suspension) | Suspension |
| 1 | 7.9 | 1.6 | 19.3 % | 8.3 |
| 2 | 3.8 | 2.2 | 39.3 % | 5.6 |
| 3 | 3.8 | 2.2 | 40.7 % | 5.4 |
| 4 | 3.8 | 4.8 | 59.3 % | 8.1 |

Table 1 (continued)

| | | | | IV · C: ng/ml |
|---|---|---|---|---|
| Urine | Supernatant | Precipitate (PPT) | Ratio (PPT/Suspension) | Suspension |
| 5 | 4.2 | 4.4 | 51.8 % | 8.5 |
| 6 | 3.7 | 2.2 | 36.7 % | 6.0 |
| 7 | 3.6 | 4.6 | 60.5 % | 7.6 |
| 8 | 5.9 | 3.1 | 35.6 % | 8.7 |
| 9 | 4.2 | 2.9 | 38.7 % | 7.5 |
| 10 | 4.8 | 2.0 | 28.6 % | 7.0 |

It is apparent from Table 1 that IV · C, a minor constituent in urine, is incorporated into the urinary precipitate. Thus, it is understood that, once precipitates are formed in urine, satisfactory assay results are not obtained unless the precipitates are well suspended prior to sampling.

With regard to the suspension used for comparison, it was found, as a result of the investigation by comparing (a) the assay case where the sample in which urinary precipitates were deposited was suspended with (b) the control case where the assay was carried out before the occurrence of precipitation in urine, that there was no affection by the deposition of urinary precipitates.

After the urine collection, the sample was divided into two, and one of them was assayed immediately after the urine collection (control) while another was subjected to formation of urinary precipitates by storage for one hour with ice cooling followed by suspension prior to assay (suspension). The results are shown in Table 2. Thus, as hereunder, a comparison was made with the case (suspension) where the sample in which urinary precipitates were formed was suspended followed by assay and the resultant data were examined.

Table 2

| | | | IV · C: ng/ml |
|---|---|---|---|
| Urine | Control | Suspension Assay | Ratio (Suspension/Control) |
| 1 | 21 | 20 | 95 % |
| 2 | 3.4 | 3.6 | 106 % |
| 3 | 2.8 | 2.9 | 104 % |
| 4 | 3.5 | 3.4 | 97 % |
| 5 | 10 | 10 | 100 % |
| 6 | 2.9 | 2.8 | 97 % |
| 7 | 3.4 | 3.5 | 103 % |
| 8 | 4.7 | 4.7 | 100 % |
| 9 | 5.6 | 5.4 | 96 % |
| 10 | 5.5 | 5.4 | 98 % |

Example 2

(1) Operating Method for TM Assay

An aliquot (50 µl) of a urine sample (if precipitated, it was suspended and then sampled) was dispensed to each test tube. Similarly a standard solution ((50 µl) was also dispensed to each test tube. Then 300 µl of an enzyme-labeled mouse anti-human TM antibody were added to the test tube followed by well mixing. Then one mouse anti-human TM monoclonal antibody-bound bead was placed in each test tube, thereby leading to an antigen-antibody reaction. The antigen-antibody reaction was carried out by allowing each test tube to stand at 15 to 30°C for one hour. After completion of the antigen-antibody reaction, the reaction solution was removed by suction, and 4.0 ml of washing solution were then added followed by removal with suction. The operation was repeated once again. Then the bead was transferred to a fresh test tube. After a coloring solution (300 μl) was added thereto, a substrate solution (300 μl) was added at predetermined intervals, then mixed and allowed to stand at 15 to 30°C for 30 minutes, thereby leading to an enzymatic reaction. A stopping solution (800 μl) was added at predetermined intervals to stop the enzymatic reaction. Absorbance at 450 nm was measured wherein water was used as a control. For the assay reagent herein, for example, a TM assay kit (TM Panasera; Fuji Chemical Industries, Ltd., Japan) can be used.

(2) Transfer of Urinary TM to Precipitate

With regard to the urine where precipitates were formed, it was centrifuged. The resultant supernatants and precipitates after the centrifugation were assayed for TM with a TM assay kit (TM Panasera; Fuji Chemical Industries, Ltd., Japan), respectively.

Remarks: The precipitates separated by the centrifugation were subjected to the assay after dissolving in a buffer of the TM assay kit (the buffer was added in the same amount as that of the urine used for the centrifugation).

The urine where precipitates were formed was suspended and then used as a control.

The results are shown in Table 3.

Table 3

| | | | | TM: FU/ml |
|---|---|---|---|---|
| Urine | Supernatant | Precipitate (PPT) | Ratio (PPT/Suspension) | Suspension |
| 1 | 5.1 | 11.8 | 77.1 % | 15.3 |
| 2 | 6.9 | 2.9 | 27.6 % | 10.5 |
| 3 | 1.0 | 9.3 | 82.3 % | 11.3 |
| 4 | 3.9 | 5.0 | 56.2 % | 8.9 |
| 5 | 8.4 | 9.2 | 51.7 % | 17.8 |
| 6 | 12.0 | 3.7 | 22.8 % | 16.2 |
| 7 | 8.3 | 4.0 | 32.0 % | 12.5 |
| 8 | 7.4 | 3.7 | 33.3 % | 11.1 |
| 9 | 9.5 | 6.0 | 36.4 % | 16.5 |
| 10 | 11.7 | 11.2 | 50.2 % | 22.3 |

It is apparent from Table 3 that TM, a minor constituent in urine, is incorporated into the urinary precipitate. Thus, it is understood that, once precipitate is formed in urine, satisfactory assay results are not obtained unless the precipitates are well suspended prior to sampling.

Example 3 Dissolution of IV・C in Urinary Precipitate

For urine where precipitates were formed, inorganic salts in the urinary precipitates were dissolved by adjusting the pH of urine to acidic or alkaline and then changes in the amount of IV・C in the urinary precipitates were investigated.

1. Investigation of Admixture with Hydrochloric Acid

To 2 ml of urine where precipitates were formed were added 50 μl of 4N hydrochloric acid (final concentration: 0.1N), and the resulting mixture was centrifuged.

The resulting precipitates were dissolved in 2 ml of a diluting solution for a standard and the amount of the IV・C incorporated into the precipitate was measured. The urine to which no hydrochloric acid was added was similarly treated and then used as a control. The results are shown in Table 4.

Table 4

| | IV · C: ng/ml | | | | |
|---|---|---|---|---|---|
| Treatment | Urine 1 | Urine 2 | Urine 3 | Urine 4 | Urine 5 |
| Hydrochloric Acid | 1.0 | 0.9 | 0.5 | 0.4 | 0.4 |
| Control | 1.1 | 0.8 | 0.5 | 0.6 | 0.5 |

It is noted from Table 4 that, for the residual precipitates after treatment with hydrochloric acid, IV · C is observed in the nearly same amount as for the precipitates from the untreated control urine sample. In other words, it is apparent that no IV · C is contained in the precipitate which is dissolved by addition of hydrochloric acid. Accordingly, it is judged that, even if the urine where precipitates are formed is acidified by treating with hydrochloric acid, it is hard to solubilize the target IV · C to be measured from the precipitates.

2. Investigation of Admixture with Acetic Acid

To 2 ml of urine where precipitates were formed were added 50 μl of 32% acetic acid (final concentration: 0.8%), the mixture was centrifuged, the resulting precipitates were dissolved in 2 ml of a diluting solution for a standard and the amount of the IV · C incorporated into the precipitates was measured. The urine to which no acetic acid was added was similarly treated and then used as a control. The results are shown in Table 5.

Table 5

| | IV · C: ng/ml | | | | |
|---|---|---|---|---|---|
| Treatment | Urine 1 | Urine 2 | Urine 3 | Urine 4 | Urine 5 |
| Acetic Acid | 0.9 | 1.1 | 0.7 | 0.4 | 0.4 |
| Control | 1.1 | 1.1 | 0.5 | 0.6 | 0.5 |

It is noted from Table 5 that, for the residual precipitates after treatment with acetic acid, IV · C is observed in the nearly same amount as for the precipitates from the untreated control urine sample. In other words, it is apparent that no IV · C is contained in the precipitate which is dissolved by addition of acetic acid. Accordingly, it is judged that, even if the urine where precipitates are formed is acidified by treating with acetic acid, it is hard to solubilize the target IV · C to be measured from the precipitates.

3. Investigation of Admixture with Ethylenediamine Tetraacetic Acid (EDTA)

To 2 ml of urine where precipitates were formed were added 100 μl of 20 mg/ml EDTA solution (final concentration: 1 mg/ml), and the mixture was centrifuged. The resulting precipitates were dissolved in 2 ml of a diluting solution for a standard and the amount of the IV · C incorporated into the precipitates was measured. The urine to which no EDTA was added was similarly treated and then used as a control. The results are shown in Table 6.

Table 6

| | IV · C: ng/ml | | | | |
|---|---|---|---|---|---|
| Treatment | Urine 6 | Urine 7 | Urine 8 | Urine 9 | Urine 10 |
| EDTA | 2.0 | 1.5 | 0.7 | 1.8 | 2.6 |
| Control | 1.8 | 1.4 | 0.5 | 1.0 | 2.3 |

It is noted from Table 6 that, for the residual precipitates after treatment with EDTA, IV · C is observed in the nearly same amount as for the precipitates from the untreated control urine sample. In other words, it is apparent that no IV · C is contained in the precipitate which is dissolved by addition of EDTA. Accordingly, it is judged that, even if the urine where precipitates are formed is acidified by treating with EDTA, it is hard to solubilize the target IV・C to be measured from the precipitates.

4. Investigation of Admixture with Ammonia

To 2 ml of urine where precipitates were formed were added 50 μl of 8% aqueous ammonia solution (final concentration: 0.2%), and the mixture was centrifuged. The resulting precipitates were dissolved in 2 ml of a diluting solution for a standard and the amount of the IV・C incorporated into the precipitate was measured. The urine to which no ammonia was added was similarly treated and then used as a control. The results are shown in Table 7.

Table 7

| | | | | | IV・C: ng/ml |
|---|---|---|---|---|---|
| Treatment | Urine 6 | Urine 7 | Urine 8 | Urine 9 | Urine 10 |
| Ammonia | 0.7 | <0.4 | <0.4 | <0.4 | <0.4 |
| Control | 1.8 | 1.4 | 0.5 | 1.0 | 2.3 |

It is noted from Table 7 that, for the residual precipitates after treatment with ammonia, the amount of IV・C is significantly reduced as compared with the case for the precipitates from the untreated control urine sample.
In other words, it is apparent that IV・C is contained in the precipitate which is dissolved by addition of ammonia. As a result of the above-mentioned investigation on dissolution of urinary precipitates by addition of hydrochloric acid, acetic acid, EDTA and ammonia, respectively, it is believed that the urinary IV・C is incorporated into the precipitates which are formed where the pH of urine is in an acidic condition.

Example 4 Dissolution of Urinary Precipitate under Alkaline Conditions

Based upon the results of Examples 1 to 3, changes in the amount of IV・C were further investigated in the precipitates when urine was made alkaline.
Thus, 6N sodium hydroxide, a 20 mg/ml EDTA-NaOH solution (pH 9.0), a 2M Tris solution and a 9% aqueous ammonia solution were each independently added to each urine where precipitates were formed, respectively, so that the pH of urine was gradually raised and finally adjusted to 8.0.
The resultant precipitates and supernatants were assayed for IV・C, respectively. As a control, untreated urine was subjected to suspension prior to assay.

1. Appearance

When each urine where precipitates were formed was admixed with 6N sodium hydroxide, a 20 mg/ml EDTA-NaOH solution (pH 9.0), a 2M Tris solution and a 9% aqueous ammonia solution, respectively, the urinary precipitates were dissolved under neutral through weakly alkaline conditions (pH 7 to less than 8). When the admixing amount was increased and the pH was made 8.0, fresh precipitates were again formed. It is noted from these results that the urinary precipitates can be dissolved when the pH of urine is adjusted to from neutral through weakly alkaline (pH 7 through 8).

2. IV・C Assay

Each of two kinds of urine where precipitates were formed was admixed with 6N sodium hydroxide, a 20 mg/ml EDTA-NaOH solution (pH 9.0), a 2M Tris solution and a 9% aqueous ammonia solution, respectively, so that the pH of urine was made 8.0. These were centrifuged and then assayed for IV・C. The results are shown in Table 8.

Table 8

| | | | | | | IV・C: ng/ml |
|---|---|---|---|---|---|---|
| Additive | | Urine 11 | | | Urine 12 | |
| | Precipitate | Supernatant | Control | Precipitate | Supernatant | Control |
| Control | 1.2 | 2.2 | 3.8 | 1.0 | 2.4 | 3.4 |

Table 8 (continued)

| Additive | Urine 11 | | | Urine 12 | | IV · C: ng/ml |
|---|---|---|---|---|---|---|
| | Precipitate | Supernatant | Control | Precipitate | Supernatant | Control |
| NaOH | 0.4 | 3.8 | 3.8 | <0.4 | 3.3 | 3.4 |
| EDTA-NaOH | 0.5 | 3.7 | 3.8 | <0.4 | 3.6 | 3.4 |
| Tris | 0.5 | 3.7 | 3.7 | <0.4 | 3.5 | 3.2 |
| $NH_3$ | 0.4 | 3.8 | 3.9 | <0.4 | 3.6 | 3.4 |

It is apparent from Table 8 that, when the pH of urine was made 8.0, the IV · C incorporated into the urinary precipitates was solubilized. When the pH of urine was adjusted to 8.0, the urinary precipitates were once dissolved and then fresh precipitates were again formed. It is apparent that no IV · C was incorporated into the precipitate which was again formed under such alkaline conditions.

Example 5 Dissolution of Urinary Precipitate under Neutral through Weakly Alkaline Conditions

As a result of the investigation on dissolution of urinary precipitates under alkaline conditions as shown in the above Example 4, it was noted that the IV · C in the urinary precipitates was dissolved under an alkaline condition. However, when the pH was 8.0 or higher, precipitates were again formed. Therefore, an investigation was further carried out on whether urinary precipitates can be dissolved under neutral through weakly alkaline conditions (pH 7 through 8). In order to adjust it to a specific pH region, a specific buffer was selected and investigated since the pH control was relatively easily achieved by only keeping the final concentration of a buffer constant and such buffers were believed to be preferred.
Each of eight kinds of urine where precipitates were formed was admixed with 0.1M phosphate buffer (pH 7.5), 1M phosphate buffer (pH 7.0), 2M HEPES buffer and 1.5M Tris · HCl buffer (pH 7.5), respectively, and their appearance was checked.
The result was that the urinary precipitates were dissolved when each of the above-mentioned buffers was added thereto. The most effective one among the above was 1.5M Tris · HCl buffer (pH 7.5). It is apparent from the results that the urinary precipitates were dissolved when the pH of urine was made neutral through weakly alkaline. It is preferred herein that buffers have a sufficient buffering ability.

Example 6 Effect by Addition of Buffer in Urinary IV · C Assay

1) Urine from Healthy Persons

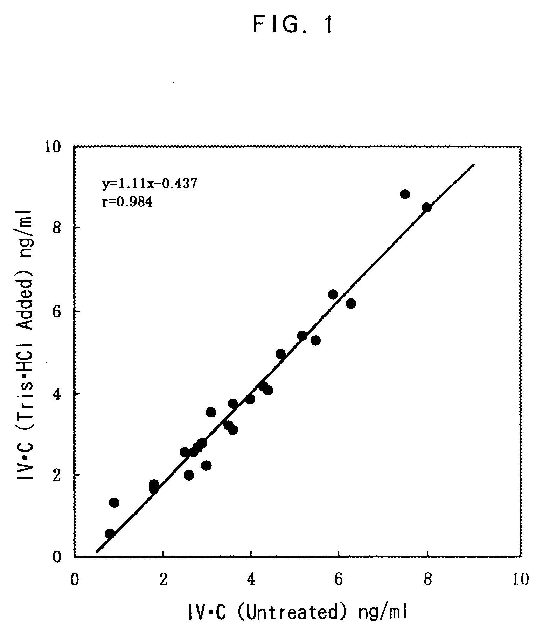

Effects by addition of Tris · HCl buffer were investigated using 1.5M Tris · HCl buffer (pH 7.5) for 25 urine samples from healthy persons wherein the frozen samples were stored. For the urine after thawing, the deposition of precipitates was noted in 16 urine samples among the 25 from healthy persons. When each of the 16 urine samples was admixed with 1.5M Tris · HCl buffer (pH 7.5) in an amount of 1/10 by volume, the precipitates were dissolved.
With regard to those 25 urine samples from healthy persons, untreated urine and Tris · HCl-admixed urine were assayed for IV · C whereupon a good correlation was obtained. Incidentally, among the untreated urine samples, those wherein precipitates were formed were subjected to suspension prior to assay.
Between the untreated urine and the Tris · HCl buffer-admixed urine, the correlation coefficient of IV · C assay values was $r = 0.984$ and the regression formula was $y = 1.11x - 0.437$  whereupon a good correlation was noted. The results are shown in Fig. 1.
It is apparent that the IV · C incorporated into the urinary precipitates can be solubilized by admixing with a Tris · HCl buffer to adjust the pH of urine to from neutral through weakly alkaline (pH 7.0 through 8.0; particularly pH 7.5) (see Fig. 1).

2) Urine from Patients Suffering from Diabetic Nephropathy

Effects by addition of Tris · HCl buffer were investigated for 100 frozen urine samples obtained from patients suffering from diabetic nephropathy. For the urine after thawing, the deposition of precipitates was noted in 60 urine samples among the 100 of the patients suffering from diabetic nephropathy. When each of the 60 urine samples was admixed with 1.5M Tris・HCl buffer (pH 7.5) in an amount of 1/10 by volume, the precipitates were dissolved in all cases.

The 60 samples where the precipitates were formed after thawing as aforementioned were used. Untreated urine and Tris・HCl buffer-admixed urine were assayed for IV・C whereupon a good correlation was obtained. Incidentally, the untreated urine was subjected to suspension prior to assay.

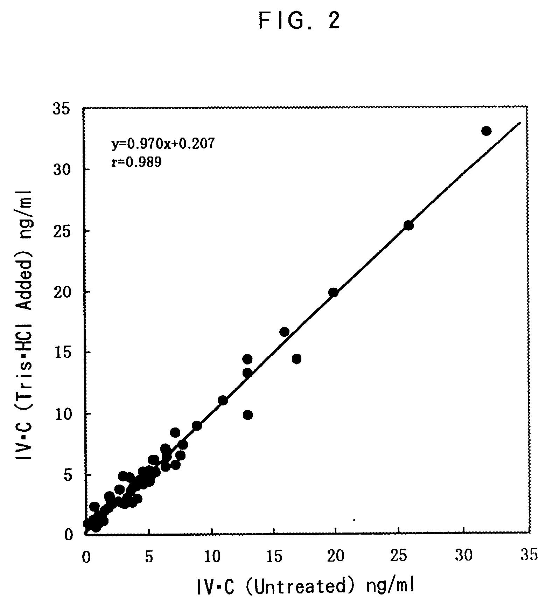

Between the untreated urine and the Tris・HCl buffer-admixed urine, the correlation coefficient of IV・C data was r = 0.989 and the regression formula was y = 0.970x + 0.207 whereupon a good correlation was obtained. The results are shown in Fig. 2.

It is apparent that the IV・C incorporated into the urinary precipitates can be solubilized by keeping the pH of urine at neutral through weakly alkaline (pH 7.0 through 8.0; particularly pH 7.5) by admixture with Tris・HCl buffer (see Fig. 2).

Example 7 Effect by Addition of Buffer in Urinary TM Assay

Four urine samples where precipitates were formed were used for investigating effects by addition of Tris・HCl buffer wherein 1.5M Tris・HCl buffer (pH 7.5) is used.

With regard to the urine where precipitates were formed, urine admixed with 1/10 volume of 1.5M Tris・HCl buffer (pH 7.5), untreated urine (subjected to suspension prior to assay), and precipitates isolated by centrifugation of the untreated urine were assayed for TM. As a result, when 1/10 volume of 1.5M Tris・HCl buffer (pH 7.5) was admixed, the precipitates were dissolved in all cases.

The TM assay results are shown in Table 9.

Table 9

| | | | TM: FU/ml |
|---|---|---|---|
| Sample | Untreated (Suspension) | Untreated (Precipitate) | Admixed with Tris・HCl |
| Urine 13 | 4.4 | 3.8 | 4.6 |
| Urine 14 | 5.6 | 4.1 | 4.8 |
| Urine 15 | 5.5 | 1.9 | 4.8 |
| Urine 16 | 8.7 | 8.7 | 8.9 |

It is apparent from Table 9 that the TM incorporated into the urinary precipitates was solubilized when 1.5M Tris・HCl buffer (pH 7.5) was added to urine to make the pH neutral through weakly alkaline (pH 7.0 through 8.0; particularly pH 7.5).

Example 8

(1) Distribution of pH of Urine.

The pH of 79 urine samples from healthy persons and 10 samples from diabetic patients was checked. The results are shown in Table 10.

Table 10

| pH | Urine from Healthy Person | | Urine from Diabetic Patient | |
|---|---|---|---|---|
| | Sample Number | % | Sample Number | % |
| pH <7.0 | 71/79 | 90 | 9/10 | 90 |
| pH $\geqq$7.0 | 8/79 | 10 | 1/10 | 10 |

The pH values (lowest-highest) with regard to the urine from healthy persons and from diabetic patients were distributed within a range of pH 5.06 through 7.52 and pH 5.60 through 7.10, respectively, and samples in the pH range of lower than 7.0 occupied 90%. As shown in Example 3, IV・C in urine is incorporated into the precipitates which are formed when the pH of urine is acidic. Accordingly, it is believed that, only when the present invention is applied, clinically significant results are obtained in about 90% of both the healthy persons and the diabetic patients without a troublesome treatment such as suspension.

(2) Frequency of Formation of Urinary Precipitate

In order to check the frequency of formation of precipitates in urine, urine immediately after collection from 64 diabetic patients was divided into two and one of them was allowed to stand at room temperature for three hours while another was allowed to stand overnight at 10°C (stored with cooling). Thereafter, the occurrence of precipitation in urine was observed.

The results are shown in Table 11. Incidentally, no precipitation was noted from urine immediately after collection.

Table 11

| Storage Condition | Number of Samples | Ratio |
| --- | --- | --- |
| Room Temp, 3 Hours | 36/64 | 56% |
| 10°C, Overnight | 49/64 | 77% |

As shown in Table 11, the formation of precipitates was noted in 56% of the samples even when allowed to stand at room temperature for three hours. In the case of storing with cooling, the formation of precipitates was noted in 77% of the samples. Since precipitates are easily formed in urine as such, it is believed that, when time is consumed from urine collection until assay or when urine samples in a cold stored state are sent to a test center, etc., an incorporation of minor constituents in urine takes place due to the formation of urinary precipitates.

Plural dispensing and pouring operations are carried out from urination until quantitative assay for each target component in urine. Thus, quantitative assays are conducted after (1) distribution from a urine-collecting cup to a transportable test tube, (2) distribution from the transportable test tube for transportation to a place where each item is assayed, (3) sampling at the assay place, etc. When the target components are incorporated into the urine precipitates upon conducting the above-mentioned dispensing, pouring, and sampling operations, sufficient suspending operation is required for each of the dispensing, pouring, and sampling operations. If non-uniformity occurs in the suspending operations, it will be impossible to assay accurately for the target component. Accordingly, when the present invention is carried out, minor constituents incorporated into the urinary precipitates are solubilized whereby it is now possible to conduct an accurate assay of target components.

(3) Lower pH Limit for Dissolving Urinary Precipitate

To urine in which precipitates were formed was added 1.5M Tris • HCl buffer (pH 7.5) stepwise and the pH where the precipitate was dissolved by the buffer addition was investigated. As a result, as shown in Table 12, the urinary precipitate was dissolved at around pH 7.0. In the urine having a lower pH, the dissolution of precipitates was observed at a relatively low pH. It is therefore believed that, in such urine, precipitates can be solubilized by adding a buffer having a low pH to the urine and that troubles caused by the formation of precipitates can be eliminated in assays.

Table 12

| Sample | pH before Treatment | pH after Dissolution of Precipitate |
| --- | --- | --- |
| 1 | 5.48 | 6.73 |
| 2 | 5.84 | 7.03 |
| 3 | 6.16 | 7.01 |
| 4 | 5.59 | 6.89 |
| 5 | 5.51 | 6.99 |
| 6 | 5.40 | 6.88 |
| 7 | 6.35 | 7.10 |

Table 12 (continued)

| Sample | pH before Treatment | pH after Dissolution of Precipitate |
|--------|---------------------|-------------------------------------|
| 8 | 5.52 | 7.00 |

(4) Changes in pH of Urine during Storage of Urine

For three types of urine, the pH value of each urine was measured (1) immediately after urine collection, (2) three days after storage with cooling and (3) three days after freezed storage. As a result, as shown in Table 13, there was almost no change in the pH in any of both (a) cooled and stored (refrigerated) and (b) freezed and stored urine samples.

Table 13

| Sample | Immediately after Urine Collection | Stored with Cooling | Freezed and Stored |
|--------|-----------------------------------|---------------------|--------------------|
| Urine 4 | 6.35 | 6.52 | 6.54 |
| Urine 5 | 6.54 | 6.55 | 6.58 |
| Urine 6 | 6.73 | 6.73 | 6.77 |

Accordingly, it is believed that the formation of precipitates during storage of urine would not be caused by changes in the pH of urine.

Example 9 Dissolution of Urinary Precipitate by Admixing with Buffer, pH 7.0

To urine wherein precipitates were formed was added a 1.5M Tris・HCl buffer (pH 7.0) stepwise and the pH where the precipitate was dissolved by the addition of buffers, the amount of the admixed buffers, and the amount of IV・C in urine (by the same assay method as in Example 1) were investigated. Incidentally, the amount of IV・C in the untreated urine was measured after the precipitated urine was suspended.
As a result, the urinary precipitates were dissolved at pH 6.6 or higher and the amount of IV・C in urine was nearly constant before and after the addition of buffers, as shown in Table 14. However, the admixed buffer necessary for dissolving the urinary precipitate was in an amount range of from 0.36 to 0.64 by volume against one volume of urine. As compared with the fact that 0.1 volume was sufficient in case where 1.5M Tris・HCl buffer (pH 7.5) was used, the buffer was needed more.

Table 14

| Sample | Untreated | | | Admixed with 1.5 M Tris・HCl Buffer, pH 7.0 | | | |
|--------|-----------|---|---|------------------------------------------|---|---|---|
| | Volume ml | pH | IV・C ng/ml | Admixed Amount ml | pH | IV・C ng/ml | Dilution Ratio Compensation IV・C ng/ml |
| Urine 1 | 7 | 6.18 | 8.4 | 2.56 | 6.73 | 6.1 | 8.3 |
| Urine 2 | 7 | 5.77 | 2.6 | 4.0 | 6.61 | 1.8 | 2.8 |
| Urine 3 | 7 | 6.03 | 4.0 | 4.5 | 6.76 | 2.6 | 4.3 |

It is apparent from the above test results that the urinary precipitates can be solubilized with 1.5M Tris・HCl buffer (pH 7.0). However, its necessary addition amount is at least 4 to 7 fold as compared with a 1.5M Tris・HCl buffer (pH 7.5). Therefore, in view of quantitatively assaying minor constituents in urine, it is necessary to get rid of any problems in the assay (for example, the sample should not be diluted too much).

Example 10 Influence of Admixture with Tris • HCl on Assay of Albumin, Transferrin, etc.

To urine samples (where urinary precipitate was formed) from patients suffering from diabetic nephropathy was added or was not added Tris • HCl buffer followed by centrifugation. The resultant supernatants were assayed for albumin, transferrin and IgG. The assay kit and method for this example are as follows:

Urinary albumin: TIA, Nitto Boseki K.K., Japan
Urinary transferrin: LTIA, Nippon DPC K.K., Japan
Urinary IgG: Plate competitive assay; BML

Each sample was treated as follows:
Each urine (6 ml) from patients suffering from diabetic nephropathy was dispensed to two test tubes and one of the test tubes was centrifuged as it was and the resulting supernatant was subjected to the assay. To another was added 0.6 ml of 1.5M Tris • HCl buffer (pH 7.5). As a result, it was confirmed that the precipitates were dissolved. The assay results are shown in Tables 15 to 17, wherein each concentration is a value calibrated for liquid volume.

Table 15

| Influence of Admixture with Tris • HCl Albumin (µg/ml) | | | |
|---|---|---|---|
| Urine | No Addition | Addition | Ratio (%) |
| 1 | 6.3 | 14.9 | 237 |
| 2 | 8.5 | 9.7 | 114 |
| 3 | 4.7 | 5.0 | 106 |
| 4 | 22.4 | 22.7 | 101 |
| 5 | 2.6 | 7.5 | 288 |
| 6 | 36.4 | 73.6 | 202 |
| 7 | 1.5 | 5.9 | 393 |
| 8 | 3.2 | 3.2 | 100 |
| 9 | 1.8 | 1.9 | 106 |
| 10 | 12.5 | 12.1 | 97 |

(a) Albumin Assay

Measured amounts of albumin in the urine to which Tris • HCl was added were 97 to 393% as compared with those in the untreated case, and it was noted that, in some cases, the measured data increased to an extent of twice or more as compared with those for the untreated case. Accordingly, it is believed that albumin is contained in the urinary precipitate.

Table 16

| Influence of Admixture with Tris • HCl Transferrin (µg/ml) | | | |
|---|---|---|---|
| Urine | No Addition | Addition | Ratio (%) |
| 1 | 16 | 47 | 294 |
| 2 | 10 | 40 | 400 |
| 3 | 0 | 14 | |
| 4 | 112 | 583 | 520 |
| 5 | 0 | 19 | |

Table 16 (continued)

| Influence of Admixture with Tris · HCl Transferrin (µg/ml) | | | |
|---|---|---|---|
| Urine | No Addition | Addition | Ratio (%) |
| 6 | 17 | 35 | 206 |
| 7 | 8 | 72 | 900 |

(b) Transferrin Assay

with regard to the measured value of transferrin in the urine to which Tris · HCl was added, there were some cases where the measured values increased as compared with those in the untreated samples. Accordingly, it is believed that transferrin is contained in the urinary precipitate.

(c) IgG Assay

Measured values of IgG in the urine to which Tris · HCl was added increased in most of the cases as compared with those in the untreated samples.

Table 17

| Influence of Admixture with Tris · HCl IgG (µg/ml) | | | |
|---|---|---|---|
| Urine | No Addition | Addition | Ratio (%) |
| 1 | 0.23 | 2.51 | 1091 |
| 2 | 1.85 | >6.4 | >346 |
| 3 | 0.47 | 2.68 | 570 |
| 4 | 3.18 | >6.4 | >201 |
| 5 | 5.37 | >6.4 | >119 |
| 6 | 0.19 | 1.62 | 853 |
| 7 | 3.19 | 4.03 | 126 |
| 8 | 1.23 | 1.73 | 141 |
| 9 | 1.96 | 5.70 | 291 |

Example 11 Influence of Admixture with Tris · HCl on $\alpha_1$MG and $\beta_2$MG

Urine samples (wherein urinary precipitates were formed) from patients suffering from diabetic nephropathy were used. To each urine sample was added or was not added Tris · HCl, followed by centrifugation. The resultant supernatants were assayed for $\alpha_1$MG and $\beta_2$MG. The assay kit and method used in this example are as follows:

$\alpha_1$-microglobulin in urine: RIA 2 antibody method
$\beta_2$-microglobulin in urine: RIA 2 antibody method

Each sample was treated as follows:
Each urine (6 ml) from patients suffering from diabetic nephropathy was dispensed to two test tubes and one of the test tubes was centrifuged, as a treatment prior to assay, as it was, and the resulting supernatant was subjected to the assay. To another was added 0.6 ml of 1.5M Tris · HCl buffer (pH 7.5). As a result, it was confirmed that the precipitates were dissolved. The assay results are shown in Tables 18 and 19, wherein each concentration is a value calibrated for liquid volume.

Table 18

| Influence of Admixture with Tris • HCl $\alpha_1$MG (mg/l) | | | |
|---|---|---|---|
| Urine | No Addition | Addition | Ratio (%) |
| 1 | 0.56 | 1.04 | 186 |
| 2 | 7.55 | 8.61 | 114 |
| 3 | 3.44 | 3.61 | 105 |
| 4 | 0.97 | 1.98 | 204 |
| 5 | 6.01 | 10.36 | 172 |
| 6 | <0.29 | 3.07 | >1059 |
| 7 | 2.25 | 3.30 | 147 |
| 8 | 0.42 | 0.48 | 114 |
| 9 | 4.14 | 3.91 | 94 |

1. $\alpha_1$-Microglobulin Assay

In most of the cases, the measured values of $\alpha_1$MG in the urine admixed with Tris • HCl increased as compared with those in the untreated case. Accordingly, it is believed that $\alpha_1$MG is contained in the precipitate.

Table 19

| Influence of Admixture with Tris • HCl $\beta_2$MG (mg/l) | | | |
|---|---|---|---|
| Urine | No Addition | Addition | Ratio (%) |
| 1 | 37 | 40 | 108 |
| 2 | 35 | 38 | 109 |
| 3 | 79 | 84 | 106 |
| 4 | 67 | 102 | 152 |
| 5 | 40 | 65 | 163 |
| 6 | 107 | 590 | 551 |
| 7 | 42 | 61 | 145 |
| 8 | 97 | 110 | 113 |
| 9 | 36 | 35 | 97 |
| 10 | 73 | 68 | 93 |

2. $\beta_2$-Microglobulin Assay

In most of the cases, the measured values of $\beta_2$MG in the urine admixed with Tris • HCl increased as compared with those in the untreated case. Accordingly, it is believed that $\beta_2$MG is contained in the precipitate.
Thus, in the assays for $\alpha_1$MG and $\beta_2$MG in urine, the addition of Tris • HCl to urine facilitates the solubilization of $\alpha_1$MG and $\beta_2$MG incorporated into the urinary precipitate (i.e., the solubilization of components in the urinary precipitate) whereby the buffer-addition is believed to be very significant as a treatment for assays of $\alpha_1$MG and $\beta_2$MG in urine.

Example 12 Influence of Centrifugal Treatment

In order to investigate the influence of the centrifugal treatment on the albumin assay, each urine sample (where urinary precipitates were formed) from patients suffering from diabetic nephropathy was divided into four. In the investigation, a combination of 4 conditions: Tris·HCl-addition and non Tris·HCl-addition, centrifugal treatment and natural sedimentation was constructed. For the assay kit and method in this example, an albumin in urine: immunoturbidimetry was used. Each sample was treated as follows:

Each suspension (5 ml) of urine samples (where a lot of precipitates were formed) was poured into four test tubes and two test tubes among them were not treated while each of other two test tubes was admixed with 0.5 ml of 1.5M Tris·HCl (pH 7.5) whereupon it was checked that the precipitates were dissolved. The samples to which no Tris·HCl buffer was added were subjected to the assays for each item (1) after they were centrifuged and (2) after the urinary precipitates were naturally sedimented. The samples to which Tris·HCl was added were subjected to the same treatment as well. The assay results are shown in Table 20.

Table 20

| Albumin in Urine uAlb : ug/ml | | | | | | | | |
|---|---|---|---|---|---|---|---|---|
| Sample | No Tris·HCl Added | | | | Tris·HCl Added | | | |
| | Centrifuged | | Naturally Sedimented | | Centrifuged | | Naturally Sedimented | |
| | Measured Value | Ratio (%) | Measured Value | Ratio (%) | Measured Value | Ratio (%) | Measured Value | Ratio (%) |
| 1 | 0.6 | 100 | 0.5 | 83 | 3.3 | 550 | 3.3 | 550 |
| 2 | 0.6 | 100 | 0.4 | 67 | 2.2 | 367 | 2.2 | 367 |
| 3 | 18.1 | 100 | 17.8 | 98 | 24.0 | 132 | 24.8 | 137 |
| 4 | 1.2 | 100 | 1.1 | 92 | 5.0 | 413 | 5.6 | 468 |
| 5 | 1.7 | 100 | 1.4 | 82 | 5.8 | 343 | 5.5 | 324 |
| 6 | 2.3 | 100 | 2.3 | 100 | 9.2 | 402 | 9.5 | 411 |
| 7 | 23.6 | 100 | 21.9 | 93 | 31.1 | 132 | 30.3 | 128 |
| 8 | 1.7 | 100 | 1.5 | 88 | 4.3 | 252 | 4.1 | 239 |
| 9 | 1832.0 | 100 | 1891.0 | 103 | 1903.0 | 104 | 1805.1 | 99 |
| 10 | 4.8 | 100 | 4.7 | 98 | 5.0 | 103 | 4.6 | 96 |
| Average | 100 % | | 90 % | | 280 % | | 282 % | |

In the group where no Tris·HCl was added, influence of pretreatments, i.e., centrifugation and natural sedimentation, for the sample on the measured values of albumin was investigated. As a result, it has been found, with regard to the influence of centrifugal treatment as the pretreatment for the sample, that a relative value to the measured values in the centrifugal treatment is 90% in average as shown in Table 20. Accordingly, it was believed that, even when either centrifugal treatment or natural sedimentation was optionally carried out as a pretreatment for the sample, the amount of components in the supernatant was not affected thereby. With regard to the influence of admixture with Tris·HCl in the assay of urinary albumin, it was suggested that urinary albumin was contained in the urinary precipitate.

Example 13 Effect of Various Buffers

1. The following various buffers were prepared and each of them was added to 3.5 ml each of three types of urine where urinary precipitates were formed whereupon the state of each urinary precipitate was observed and IV·C values in the urine samples were measured. When there was still a precipitate, IV·C values in the supernatant were measured.

(1) 0.5M $Na_2HPO_4$-$KH_2PO_4$ buffer (pH 6.5)
(2) 0.5M citric acid-$Na_2HPO_4$ buffer (pH 6.5)
(3) 1M imidazole-HCl buffer (pH 6.5)
(4) 1M HEPES buffer (pH 6.8)
(5) 1M TES (N-trismethyl-2-aminoethanesulfonic acid) buffer (pH 6.8)

The result was that, when a buffer was added, the urinary precipitates were partially or wholly dissolved and all the IV $\cdot$ C values in the urine samples increased as compared with the case where no buffer was added (Table 21-1). It was therefore confirmed that the IV $\cdot$ C in the urinary precipitate was solubilized by addition of the buffer. Thus, those buffers are effective for dissolving urinary precipitates and can solubilize IV $\cdot$ C (one of urine minor constituents) in urinary precipitates.

Table 21-1

| Urine | Buffer | None | (1) | (2) | (3) | (4) | (5) |
|---|---|---|---|---|---|---|---|
| 1 | Amount Added (ml) | - | 3.5 | 3.0 | 3.5 | 3.5 | 2.5 |
| | pH after Addition | 5.99 | 6.50 | 6.48 | 6.49 | 6.50 | 6.60 |
| | Changes in Precipitate | - | Dec. | Dec. | Dec. | Dec. | Dec. |
| | IV $\cdot$ C in Supernatant (ng/ml) | 2.2 | 3.6 | 3.6 | 3.8 | 2.6 | 3.9 |
| 2 | Amount Added (ml) | - | 4.5 | 4.0 | 4.5 | 5.0 | 1.0 |
| | pH after Addition | 6.21 | 6.56 | 6.54 | 6.57 | 6.65 | 6.72 |
| | Changes in Precipitate | - | Dec. | Dec. | Dec. | Dec. | Dec. |
| | IV $\cdot$ C in Supernatant (ng/ml) | 3.9 | 5.3 | 5.1 | 5.3 | 4.1 | 4.3 |
| 3 | Amount Added (ml) | - | 4.0 | 4.0 | 3.0 | 4.0 | 3.0 |
| | pH after Addition | 5.98 | 6.57 | 6.55 | 6.50 | 6.58 | 6.64 |
| | Changes in Precipitate | - | Dis. | Dis. | Dis. | Dis. | Dis. |
| | IV $\cdot$ C in Supernatant (ng/ml) | 7.2 | 8.3 | 8.2 | 7.8 | 8.4 | 8.4 |

Dis. (dissolved): Urinary precipitates were wholly dissolved
Dec. (decreased): Urinary precipitates decreased due to their partial dissolution

2. The following various buffers were prepared and each of them was added to 3.5 ml each of three types of urine where urinary precipitates were formed whereupon the state of each urinary precipitate was observed and IV $\cdot$ C values in the urine samples were measured. When there was still a precipitate, IV $\cdot$ C values in the supernatant were measured.

(1) 0.5M boric acid-KCl-NaOH buffer (pH 8.2)
(2) 0.5M $Na_2HPO_4$-$KH_2PO_4$ buffer (pH 8.0)
(3) 0.5M citric acid-$Na_2HPO_4$ buffer (pH 8.0)
(4) 0.05M barbital Na-HCl buffer(pH 8.2)
(5) 1M imidazole-HCl buffer (pH 7.8)
(6) 1M HEPES buffer (pH 8.2)
(7) 1M TES buffer (pH 8.2)

The result was that, when a buffer was added, the urinary precipitates were partially or wholly dissolved. In some of the samples, white precipitates were again formed almost together with the dissolution of the urinary precipitate. All the IV $\cdot$ C values in the urine samples greatly increased as compared with the case where no buffer was added (Table 21-2). It was therefore confirmed that the IV $\cdot$ C in the urinary precipitate was solubilized by addition of the buffer. Thus, those buffers are effective for dissolving urinary precipitates and can solubilize IV $\cdot$ C (one of minor constituents) in urinary precipitates.

Table 21-2

| Urine | Buffer | None | (1) | (2) | (3) | (4) | (5) | (6) | (7) |
|---|---|---|---|---|---|---|---|---|---|
| 1 | Amount Added (ml) | - | 3.0 | 3.0 | 3.0 | 3.5 | 1.5 | 2.0 | 1.0 |
| | pH after Addition | 5.99 | 7.86 | 7.54 | 7.45 | 6.96 | 7.72 | 7.83 | 7.85 |
| | Changes in Precipitate | - | W. PPT | W. PPT | W. PPT | Dec. | W. PPT | W. PPT | W. PPT |
| | IV·C in Supernatant (ng/ml) | 2.2 | 5.4 | 5.8 | 5.4 | 4.6 | 5.0 | 4.9 | 5.3 |
| | Ratio of IV·C Values (%) | 44 | 96 | 104 | 93 | 82 | 98 | 94 | 98 |
| 2 | Amount Added (ml) | - | 3.0 | 4.5 | 2.5 | 3.5 | 1.5 | 1.5 | 1.0 |
| | pH after Addition | 6.21 | 8.00 | 7.74 | 7.53 | 7.66 | 7.79 | 7.83 | 7.91 |
| | Changes in Precipitate | - | W. PPT | W. PPT | W. PPT | Dec. | W. PPT | W. PPT | W. PPT |
| | IV·C in Supernatant (ng/ml) | 3.9 | 4.3 | 5.3 | 5.3 | 4.8 | 5.6 | 5.4 | 5.8 |
| | Ratio of IV·C Values (%) | 72 | 80 | 96 | 96 | 96 | 110 | 106 | 105 |

Table 21-2 (continued)

| Urine | Buffer | None | (1) | (2) | (3) | (4) | (5) | (6) | (7) |
|---|---|---|---|---|---|---|---|---|---|
| 3 | Amount Added (ml) | - | 1.0 | 3.0 | 1.0 | 3.0 | 1.0 | 1.5 | 1.0 |
| | pH after Addition | 5.98 | 7.32 | 7.70 | 7.42 | 7.44 | 7.72 | 7.78 | 7.88 |
| | Changes in Precipitate | - | Dis. | W. PPT | Dis. | Dis. | Dis. | Dis. | Dis. |
| | IV·C in Supernatant (ng/ml) | 7.2 | 8.5 | 8.5 | 8.2 | 8.5 | 7.9 | 8.4 | 8.2 |
| | Ratio of IV·C Values (%) | 88 | 100 | 102 | 100 | 100 | 100 | 100 | 100 |

Dis. (dissolved) : Urinary precipitates were wholly dissolved
Dec. (decreased) : Urinary precipitates decreased due to their partial dissolution
W. PPT (white precipitate) : White precipitates were formed again almost togeter with dissolution of all urinary precipitates
Ratio of IV·C Values (%) : Ratio of IV·C value determined from the supernatant to IV·C value determined from the suspension

Example 14 pH Range of Urine upon Dissolution of Urinary Precipitate

A 1.5M Tris·HCl buffer (pH 9.5) was added to three kinds of urine samples where urinary precipitates were formed to adjust the pH of urine to about 6.5 or 8.2. The amounts of IV·C in the urine before and after the addition of buffer were measured. The results are given in Table 22.

The result was that, in any of the cases where the pH of urine was made 6.5 and 8.2, the clear decrease or dissolution of urinary precipitates was visually noted. Although there was no big difference among IV·C values (A) determined from the suspensions at any pH, the ratio of IV·C value (B) determined from the supernatant to (A) was higher at any pH than that in the case where no buffer was added. Therefore, it has been confirmed that IV·C in the urinary precipitates can be solubilized when pH is made 6.5 or 8.2.

Accordingly, it is apparent that the pH of urine which is effective for dissolving urinary precipitates is at least 6.5 through 8.2.

Table 22

| Urine No. | No Tris-Added (Original Urine) | | | | Tris Added to Make pH 6.5 | | | | | Tris Added to Make pH 8.2 | | | | |
|---|---|---|---|---|---|---|---|---|---|---|---|---|---|---|
| | pH | IV·C (ng/ml) | | | PPT | pH | IV·C (ng/ml) | | | PPT | pH | IV·C (ng/ml) | | |
| | | Suspension (A) | Supernatant (B) | Ratio (%) | | | Suspension (A) | Supernatant (B) | Ratio (%) | | | Suspension (A) | Supernatant (B) | Ratio (%) |
| 4 | 5.33 | 5.8 | 4.3 | 74 | Dec. | 6.47 | 5.6 | 4.6 | 82 | None | 8.18 | 5.7 | 5.7 | 100 |
| 5 | 5.77 | 5.8 | 4.3 | 74 | Dec. | 6.47 | 5.5 | 4.9 | 89 | W.P. | 8.21 | 5.6 | 5.7 | 102 |
| 6 | 5.53 | 5.6 | 3.7 | 66 | Dec. | 6.51 | 6.2 | 5.0 | 81 | W.P. | 8.22 | 6.2 | 6.2 | 100 |

PPT: Precipitate
Dec.: Decreased
W.P.: White precipitates were formed

Example 15 Vessel Containing an Agent for Treating a Urine Sample

A container (or vessel) which (a) contains the urine sample-treating agent of the present invention and (b) has

27

the capability of conducting required treatments, together with the collection of urine is provided. A typical container is a test tube which is used for urine assays. The shape of the test tube includes (1) a round bottom and (2) a Spitz (which has a pointed or thin end). An example of the urine-collecting tube (urine-sample receptacle) according to the present invention is a round-bottom test tube (made of PET) to which each 0.5 ml of 1.5M Tris・HCl (pH 7.5) is dispensed followed by lyophilization. In actual use, urine is added to said PET test tube up to a line of 5 ml followed by mixing whereupon it is possible to solubilize analytes to be measured which are contained in the urinary precipitate.

Industrial Applicability

In accordance with the present invention, the pH of urine wherein precipitates are formed is made weakly acidic through weakly alkaline, such as pH 6.5 through 8.0, so that minor constituents incorporated into the precipitate can be solubilized whereupon the minor constituents in the urine can be correctly assayed. In addition, when the pH of collected urine is made weakly acidic through weakly alkaline, such as pH 6.5 through 8.2, then unfavorable influence in immunoassays, biochemical assays, etc. can be prevented or excluded whereupon it is possible to obtain a significant clinical test result accurately and easily. It is also possible to transport urine samples in a substantially stable state in view of conducting an immunoassay or biochemical assay or to store them for a long period similarly to the transportation.

Since a great large number of urine samples can be correctly subjected to sampling in a facile manner even on a mass scale, it is possible to use a cooled or freezed (even stored) urine as a sample for assays which are mostly conducted in test departments, test centers, etc. having various kinds of measuring instruments and reagents. A large number of urine samples can be subjected to sampling correctly and efficiently in a labor-saving manner herein. The instant technology is suitable for automated operations as well whereby clinically significant data can be obtained easily and correctly. In conducting a quantitative assay of minor constituents in urine incorporated into the urinary precipitate, the instant art of the present invention can be utilized wherein the minor constituents incorporated into the urinary precipitate can be effectively solubilized.

Regardless of the fact whether or not a precipitate is formed in the urine sample, sampling of the urine sample suitable for immunoassays, biochemical assays, etc. is possible and, in addition, said assaying operation can be automated preferably whereupon clinically significant results can be obtained easily and accurately.

**Claims**

1. An agent for treating a urine sample, which comprises, as an effective component, a buffer in the pH of 6.5 or higher.

2. The urine sample-treating agent according to claim 1 which comprises, as an effective component, a buffer in the pH range of from 6.5 through 9.5.

3. The urine sample-treating agent according to claim 1 or 2 which comprises, as an effective component, a buffer in the pH range of from 6.5 through 8.2.

4. The urine sample-treating agent according to any of the claims 1 to 3 which comprises, as an effective component, a buffer in the pH range of from 7.4 through 7.6.

5. The urine sample-treating agent according to any of claims 1 to 4 in which the buffer is a member selected from the group consisting of phosphate buffer, N-tris(hydroxymethyl)methyl-2-aminoethanesulfonic acid (TES) buffer, N-(2-hydroxyethyl)piperazine-N'-ethanesulfonic acid (HEPES) buffer, tris(hydroxymethyl)aminomethane (Tris)-HCl buffer, borate buffer, citrate buffer, barbital buffer and imidazole-HCl buffer.

6. An agent for treating a urine sample, which comprises the property of solubilizing at least part of precipitates formed in urine.

7. The urine sample-treating agent according to claim 6 which comprises the property of solubilizing minor constituents which are or may be potentially incorporated into precipitates formed in the urine sample and are analytes to be assayed.

8. The urine sample-treating agent according to claim 6 or 7 in which the precipitate formed in the urine sample occurs during the storage of the urine sample.

9. The urine sample-treating agent according to any of claims 6 to 8 in which the precipitate formed in the urine sample occurs during the storage of urine in the pH of lower than 7.0.

10. The urine sample-treating agent according to any of claims 6 to 9 in which the "solubilizing" is carried out by increasing the pH of urine with the buffer according to any of claims 1 to 5.

11. The urine sample-treating agent according to any of claims 6 to 10 in which the "solubilizing" is carried out by adding tris(hydroxymethyl)aminomethane (Tris)-HCl buffer to urine.

12. The urine sample-treating agent according to any of claims 6 to 11 in which the minor constituent is selected from the group consisting of urinary transferrin, urinary growth hormone, urinary IgG, urinary albumin, urinary chorionic gonadotropin, urinary $\alpha_1$-microglobulin, urinary $\beta_2$-microglobulin, various types of collagen, proline hydroxylase, laminin, matrix metalloproteinases (MMPs), tissue inhibitors of metalloproteinase (TIMPs) and thrombomodulin.

13. The urine sample-treating agent according to any of claims 6 to 12 in which the minor constituent is selected from the group consisting of type IV collagen and thrombomodulin.

14. An agent for treating a urine sample, which comprises the property of making the pH of urine 6.5 or higher.

15. The urine sample-treating agent according to claim 14 which comprises the property of making the pH of urine from 6.5 through 8.2.

16. The urine sample-treating agent according to claim 14 or 15 which comprises the property of making the pH of urine from 7.4 through 7.6.

17. The urine sample-treating agent according to any of claims 14 to 16 which the property is achieved by (i) increasing the pH of urine or (ii) maintaining a predetermined pH value in urine, with the buffer according to any of claims 1 to 5.

18. The urine sample-treating agent according to any of claims 14 to 17 which the property is achieved by adding tris(hydroxymethyl)aminomethane (Tris)-HCl buffer to urine.

19. The urine sample-treating agent according to any of claims 14 to 18 in which urine is subjected to the "solubilizing" according to any of claims 8 to 13, thereby (i) substantially preventing the formation of precipitates occurring in a fresh urine from having an adverse effect on assays for target analytes, or (ii) substantially eliminating a disturbance caused by precipitates in a stored urine in view of the assays.

20. The urine sample-treating agent according to any of claims 14 to 19 which has the property (i) of preventing the formation of precipitates occurring during the storage of a fresh urine or (ii) of solubilizing precipitates in a stored urine.

21. A container for a urine sample, which contains the urine sample-treating agent according to any of claims 1 to 20 and is in a form ready to use.

22. The container according to claim 21 in which the urine sample-treating agent is contained in the form of a liquid composition.

23. The container according to claim 21 in which the the urine sample-treating agent is contained in the form of readily soluble solids;

24. The container according to claim 23 in which the urine sample-treating agent is contained in the form of lyophilized solids.

25. A method for treating a urine sample, which comprises treating urine with the urine sample-treating agent according to any of claims 1 to 20.

26. The method according to claim 25 which comprises

(a) treating urine with the urine sample-treating agent, and

(b) (i) solubilizing at least part of precipitates usually formed in the urine sample or (ii) suppressing or preventing at least partially the formation of said precipitates, for the purpose of having substantially no adverse effect on assays.

27. An assay for a urine sample, which comprises

(a) treating urine with the urine sample-treating agent according to any of any of claims 1 to 20, and
(b) then using the treated urine as a sample for assays.

28. The assay according to claim 27 which comprises

(a) treating urine with the urine sample-treating agent so as to (i) solubilize at least part of precipitates usually formed in the urine sample or (ii) suppress or prevent at least partially the formation of said precipitates, for the purpose of having substantially no adverse effect on assays, and
(b) then assaying said treated urine sample for a target analyte.

29. The assay according to claim 27 or 28 in which, in the assay of urine samples, sampling is made and target analytes are assayed without subjecting the urine sample to suspension (or agitation).

30. The assay according to claims 27 to 29 in which the target analyte is selected from the group consisting of type IV collagen and thrombomodulin and urine is used as a sample.

31. The assay according to the claims 27 to 30 which comprises automatically sampling the urine and assaying for a target analyte.

32. A kit which is a set comprising a combination of

(1)

(i) a reagent for the assay of human type IV collagen containing an anti-human type IV collagen monoclonal antibody or
(ii) a reagent for the assay of human thrombomodulin containing an anti-human thrombomodulin monoclonal antibody, with

(2)

(a) the urine sample-treating agent according to any of claims 1 to 20 or
(b) the container according to any of claims 21 to 24.

33. An assay reagent for (i) human type IV collagen or (ii) human thrombomodulin wherein (i) the human type IV collagen assay reagent contains an anti-human type IV collagen monoclonal antibody and (ii) the human thrombomodulin assay reagent contains an anti-human thrombomodulin monoclonal antibody, which comprises, as a constituent element,

(a) the urine sample-treating agent according to any of claims 1 to 20, or
(b) the container according to any of claims 21 to 24.

F I G.  1

FIG. 2

## INTERNATIONAL SEARCH REPORT

| International application No. |
| --- |
| PCT/JP97/04885 |

### A. CLASSIFICATION OF SUBJECT MATTER

Int. Cl⁶ G01N33/50, 33/53

According to International Patent Classification (IPC) or to both national classification and IPC

### B. FIELDS SEARCHED

Minimum documentation searched (classification system followed by classification symbols)

Int. Cl⁶ G01N33/48-98

Documentation searched other than minimum documentation to the extent that such documents are included in the fields searched

| | |
| --- | --- |
| Jitsuyo Shinan Koho | 1926 – 1998 |
| Kokai Jitsuyo Shinan Koho | 1971 – 1995 |
| Toroku Jitsuyo Shinan Koho | 1994 – 1998 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| Y | JP, 7-110328, A (Hitachi, Ltd.), April 25, 1995 (25. 04. 95), Claims; Par. No. (0008) & EP, 656540, A2 & US, 5585469, A | 1 – 33 |
| Y | JP, 4-502363, A (Beinbridge Laboratories Inc.), April 23, 1992 (23. 04. 92), Claims; Examples & EP, 664453, A2 & WO, 90/07116, A | 1 – 33 |
| Y | Supervised and edited by Keisuke Fujita "Guide to Biochemical Experiments and Tests in the Medical Field (in Japanese)" (1982) Hirokawa Shoten, pages 106, 107 | 1 – 33 |

☐ Further documents are listed in the continuation of Box C.　　☐ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| January 30, 1998 (30. 01. 98) | February 10, 1998 (10. 02. 98) |

| Name and mailing address of the ISA/ | Authorized officer |
| --- | --- |
| Japanese Patent Office | |
| Facsimile No. | Telephone No. |

Form PCT/ISA/210 (second sheet) (July 1992)